# EUROPÄISCHE PATENTANMELDUNG

(11) **EP 0 940 399 A1**
(43) Veröffentlichungstag der Anmeldung: **08.09.1999**
(21) Anmeldenummer: 99101903.5
(22) Anmeldetag: 29.01.1999
(51) Int. Cl.: C07D 321/00

(54) **Verfahren zur Herstellung von makrocyclischen Estern**

(30) Priorität: 03.03.1998 DE 19808844
(71) Anmelder: HÜLS AKTIENGESELLSCHAFT, 45764 Marl (DE)
(72) Erfinder: Köhler, Günther Dr., 45770 Marl (DE); Feld, Marcel Dr., 51147 Köln (DE); Osterholt, Clemens, 46286 Dorsten (DE); Metz, Josef Dr., 45770 Marl (DE)
(74) Vertreter: Olbricht, Gerhard, Dr.

(57) **Zusammenfassung**

Die Erfindung betrifft ein Verfahren zur Herstellung von makrocyclischen Estern der allgemeinen Formel 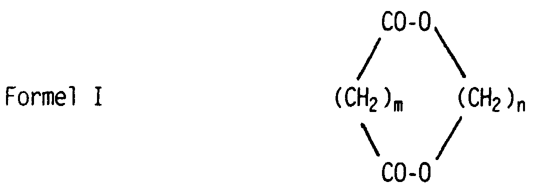 in der m die Bedeutung einer ganzen Zahl von 6 bis 14 hat und n für eine ganze Zahl von 2 bis 6 steht, wobei man einen Dicarbonsäure-bis(glykol)ester der allgemeinen Formel

HO-(CH₂)ₙ-O-CO-(CH₂)ₘ-CO-O-(CH₂)ₙ-OH, Formel II

in der m und n die oben angegebenen Bedeutungen haben,
(b) gegebenenfalls bis zu 100 Gew.-%, bezogen auf den Ester II, von hinsichtlich ihrer Bausteine dem Ester II entsprechenden Estern der allgemeinen Formeln

   H[O-(CH₂)ₙ-O-CO-(CH₂)ₘ-CO]ₓ-O-(CH₂)ₙ-OH Formel III

   H[O-(CH₂)ₙ-O-CO-(CH₂)ₘ-CO]ₓ-OH Formel IV

   und/oder

   HO-CO-(CH₂)ₘ-CO-[O-(CH₂)ₙ-O-CO-(CH₂)ₘ-CO]ₓ-OH, Formel V

   in denen m und n die zuvor angegebenen Bedeutungen haben und x für eine ganze Zahl >1 steht, sowie
(c) ein Glykol der allgemeinen Formel

   HO-(CH₂)ₙ-OH Formel VI

   in der n denselben numerischen Wert wie in den Formeln I bis V hat, in der 1- bis 50-fachen molaren Menge, bezogen auf den Ester II und die Dicarbonsäurebausteine der Esters III bis V, und
(d) ein inertes hochsiedendes Reaktionsmedium in der 0,1 bis 20-fachen Gewichtsmenge, bezogen auf die Summe der Gewichtsmengen der Ester II und III bis V, in Gegenwart
(e) eines Katalysators
in einem Verdampfer mit großer Oberfläche auf Temperaturen von 150 bis 350°C bei einem vermindertem Druck von etwa 0,1 bis etwa 500 mbar erhitzt, wodurch unter Abspaltung von Glykol der makrocyclische Ester I entsteht, der zusammen mit Glykol IV abdestilliert und durch Kondensation gewonnen wird.

## Beschreibung

Die Erfindung betrifft ein Verfahren zur Herstellung makrocyclischer, vorzugsweise 12- bis 20-gliedriger Ester der allgemeinen Formel 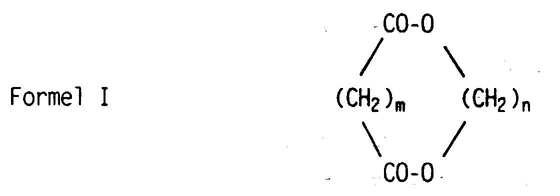 in der m die Bedeutung einer ganzen Zahl von 6 bis 14 hat und n für eine ganze Zahl von 2 bis 12 steht, durch Cyclisierung von Dicarbonsäure-bis(glykol)estern (oder Dicarbonsäure-bis(hydroxyalkyl)estern) der allgemeinen Formel

HO-(CH₂)ₙ-O-CO-(CH₂)ₘ-CO-O-(CH₂)ₙ-OH Formel II

wobei m und n die oben angegebene Bedeutung haben.

Cyclische Ester dieser Art und insbesondere das formal aus den monomeren Bausteinen Brassylsäure und Ethylenglykol gebildete cyclische Ethylenbrassylat haben in der Parfümindustrie als Duftbestandteil mit Ambra- oder Moschusnote bzw. als Fixateur in Riechstoffmischungen eine herausragende Bedeutung.

Es ist bekannt, makrocyclische Ester durch cyclisierende Depolymerisation oligomerer oder polymerer Glykolester entsprechender Dicarbonsäuren zu gewinnen. Üblicherweise wird die Depolymerisation bei hohen Temperaturen und unter vermindertem Druck so durchgeführt, daß die dabei gebildeten Zielprodukte abdestillieren und durch Kondensation gewonnen werden können. Die cyclisierende Depolymerisation ist z.B. in J.Am.Chem.Soc. **57** (1935), 929-34 und in US-A-4,175,321 beschrieben.

Es ist weiter aus US-A 4 709 058, JA-AS 55-120 581 und DE 32 25 341 bekannt, daß bei der Herstellung von makrocyclischen Estern durch cyclisierende Depolymerisation die Mitverwendung von inerten hochsiedenden Reaktionsmedien von Vorteil ist. Ein wesentliches Problem bei jeder cyclisierenden Depolymerisation besteht darin, daß unter den Reaktionsbedingungen auch höhermolekulare Ester gebildet werden können, indem Oligomere oder Polymere mit endständigen Carboxylgruppen mit anderen Oligomeren oder Polymeren, die endstandige Hydroxylgruppen tragen, unter Abspaltung von Wasser polykondensieren oder indem Oligomere oder Polymere mit endständigen Hydroxyalkylgruppen unter Glykolabspaltung polykondensieren. Die erwühschte intramolekulare Bildung der makrocyclischen mohomeren Zielprodukte wird also von einer unerwünschten intermolekularen Bildung linearer, reaktionsträger hochpolymerer Ester begleitet. Dies aber vermindert nicht nur die Ausbeute an Zielprodukt, sondern wirft auch erhebliche verfahrenstechnische Probleme auf.

Bei den bisher beschriebenen Verfahren wird die Reaktion im allgemeinen batchweise oder semikontinuierlich durchgeführt. Dies ist bei einer Synthese in kleiner Größenordnung etwa im Labor- oder Technikumsmaßstab, völlig unproblematisch. Die Übertragung des Verfahrens in den technischen Maßstab wirft jedoch Probleme auf, wobei insbesondere eine batchweise oder kontinuierliche Reaktionsführung im Rührkessel beträchtliche Nachteile aufweist. Durch die gebildeten höhermolekularen Produkte sinkt nämlich die Wärmeleitfähigkeit des Reaktionsgemisches, während gleichzeitig die Viskosität ansteigt. Dadurch wird das Abdestillieren der monomeren Zielprodukte erschwert, was wiederum die Bildung höhermolekularer Produkte begünstigt. Deren Anteil steigt also an, wodurch in zunehmendem Maße Sumpfprodukt entsteht, das entsorgt werden muß. Wird die Reaktion nicht rechtzeitig abgebrochen, ist sogar ein Erstarren des gesamten Kesselinhalts möglich.

Darüber hinaus ist es technisch nicht einfach, die für die cyclisierende Depolymerisation erforderliche Energiemenge in einen Rührkessel einzubringen und zugleich dem Zielprodukt sowie dem zwangsläufig mit dem Zielprodukt abdestillierenden gebildeten und gegebenenfalls zusätzlich eingebrachten Glykol eine möglichst große Verdampfungsfläche zu bieten.

Dem wird nach der europäischen Patentanmeldung 929 07 653.7 durch die Verwendung eines speziellen, horizontalen Dünnschichtverdampfers Rechnung getragen, der jedoch bei sehr hohen Temperaturen von >300°C und mit praktisch unverdünntem polymerem Einsatzprodukt betrieben werden muß und bei dem die Probleme durch Bildung hochpolymerer Produkte besonders gravierend sein können.

Die genannten Probleme werden durch das erfindungsgemäße Verfahren auf einfache, vorteilhafte und technisch leicht realisierbare Weise gelöst, Gegenstand der Erfindung ist ein Verfahren zur Herstellung von makrocyclischen, vorzugsweise 12 bis 20 Ringglieder umfassenden Estern der allgemeinen Formel 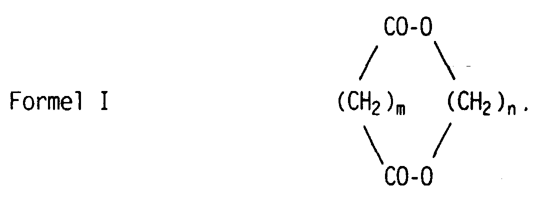 in der m die Bedeutung einer ganzen Zahl von 6 bis 14 hat und n für eine ganze Zahl von 2 bis 12 steht, durch Cyclisierung von Dicarbonsäure-bis(glykol)estern der allgemeinen Formel

HO-(CH₂)ₙ-O-CO-(CH₂)ₘ-CO-O-(CH₂)ₙ-OH, Formel II

in der m und n die für die Formel I angegebenen Bedeutungen haben, wobei man
(a) einen Dicarbonsäure-bis(glykol)ester II.
(b) gegebenenfalls bis zu 100 Gew.-%, bezogen auf den Ester II, von hinsichtlich ihrer Bausteine dem Ester II entsprechenden Estern der allgemeinen Formel

   H[O-(CH₂)ₙ-O-CO-(CH₂)ₘ-CO]ₓ-O-(CH₂)ₙ-OH, Formel III

   H[O-(CH₂)ₙ-O-CO-(CH₂)ₘCO]ₓ-OH Formel IV

   und/oder

   HO-CO-(CH₂)ₘ-CO-[O-(CH₂)ₙ-O-CO-(CH₂)ₘ-CO]ₓ-OH. Formel V

   in denen m und n denselben numerischen Wert wie in den Formeln I und II haben und x für eine ganze Zahl >1, vorzugsweise von 2 bis 10 steht,
(c) ein Glykol der allgemeinen Formel

   HO-(CH₂)ₙ-OH Formel VI

   in der n denselben numerischen Wert wie in den Formeln I bis V hat, in der 1- bis 50-fachen, vorzugsweise in der 2- bis 20-fachen molaren Menge Menge. bezogen auf den Ester II und die Dicarbonsäurebausteine der Ester III bis V. und
(d) ein inertes hochsiedendes Reaktionsmedium in der 0,1 bis 20-fachen, vorzugsweise in der 1- bis 15-fachen und insbesondere in der 2 bis 10-fachen Gewichtsmenge. bezogen auf die Summe der Gewichtsmengen der Ester II bis V, in Gegenwart
(e) eines Katalysators
in einem Verdampfer mit großer Oberfläche auf Temperaturen von 150 bis 350°C. vorzugsweise von 180 bis 300°C und insbesondere von 200 bis 280°C, bei einem vermindertem Druck von etwa 0,1 bis etwa 500 mbar, vorzugsweise von 0,5 bis 100 mbar erhitzt, wodurch unter Abspaltung von Glykol der makrocyclische Ester I entsteht. der zusammen mit Glykol IV abdestilliert und durch Kondensation gewonnen wird.

Überraschenderweise erhält man bei dem erfindungsgemäßen Verfahren Ausbeuten an makrocyclischem Ester I von >90 % d.Th. trotz der Anwesenheit von überschüssigem Glykol IV, das das Umesterungsgleichgewicht in Richtung auf den Dicarbonsäurebis-(glykol)ester II verschiebt, also die Cyclisierungsreaktion zurückdrängt.

Das erfindungsgemäße Verfahren wird vorteilhaft kontinuierlich durchgeführt, wobei das inerte hochsiedende Medium, das zunächst den Katalysator und nicht umgesetzte Ester II bis V enthält, im Kreis geführt wird. Bevorzugt wird das kontinuierliche Verfahren so durchgeführt, daß man die Lösung eines Dicarbonsäure-bis(glykol)esters II in dem entsprechenden Glykol, die den Katalysator und gegebenenfalls Ester III bis V enthält, in das rückgeführte inerte hochsiedende Medium einbringt und die entstehende Mischung in die Verdampfungszone einführt, die zugleich Reaktionszone ist und aus der der gebildete makrocyclische Ester I und Glykol VI abdestillieren. Die Edukte, d.h. die Ester II bis V, werden in nur einem Durchgang weitgehend umgesetzt. Bei optimaler Fahrweise verbleiben daher in der Regel nur 1 bis 10 Gew.-% Edukt, bezogen auf das inerte hochsiedende Medium, unverdampft zurück und werden nach Anreicherung mit frischem Edukt und Glykol in die Verdampfungszone zurückgeführt. Die Verweilzeit in dem Verdampfer mit großer Oberfläche beträgt pro Durchgang vorteilhat 0,5 bis 10 Minuen. Sie wird durch die Leistung der Pumpe geregelt, die den Kreislauf bewirkt. Nach Erreichen des stationären Zustandes werden bis zu 100% der Edukte in das Zielprodukt I überführt. Im Verlauf einer längeren Produktionskampagne, die eine Anlaufphase und den stationären Zustand umfaßt, werden somit - je nach Länge der Kampagne - weit über 95% der Einsatzprodukte zum Zielprodukt I umgesetzt.

Die Ester II bis V leiten sich von Dicarbonsäuren und Glykolen (oder Diolen) ab. Geeignete Dicarbonsäuren haben beispielsweise 2 bis 20, vorzugsweise 4 bis 12 Kohlenstoffatome zwischen den Carboxylgruppen. Beispiele hierfür sind u.a. Bernsteinsaure, Adipinsäure, Korksäure, Sebacinsäure (1,10-Decandisäure), 1,12-Dodecandisäure und Brassylsäure (1,3-Tridecandisäure). Von den geeigneten Diolen, die z.B. 2 bis 12 Kohlenstoffatome zwischen den Hydroxylgruppen haben können, seien z.B. Ethylenglykol, Ethylendiglykol, 1,3-Propandiol, 1,4-Butandiol, 1,6-Hexandiol, 1,8-0ctandiol und 1,12-Dodecandiol genannt.

Eine für die Durchführung des erfindungsgemäßen Verfahrens geeignete glykolische Lösung der Ester II und III bis V kann beispielsweise unter Verwendung üblicher Veresterungs- bzw. Umesterungskatalysatoren durch direkte Veresterung der Dicarbonsäure mit dem Glykol VI, durch Umesterung eines Dicarbonsäuredialkylesters eines aliphatischen, niedermolekularen Alkohols mit vorzugsweise 1 bis 6 Kohlenstoffatomen mit dem Glykol VI unter Abspaltung des aliphatischen niedermolekularen Alkohols oder durch Depolymerisation eines hochpolymeren Esters der Formeln III bis V, in der x z.B. = >10 ist, mit überschüssigem Glykol IV hergestellt werden. Im letzteren Fall werden also hochpolymere Ester (x = >10) zu einem Gemisch aus monomerem Ester II und höhermolekularen Estern III (x = 2 bis 10) abgebaut (die Entstehung von höhermolekularen Estern IV und V mit freien Carboxylgruppen ist wegen des Glykolüberschusses nicht begünstigt). In allen Fällen wird die Bildung des Dicarbonsäure-bis(glykol)esters II mit zunehmendem Überschuß an Glykol VI begünstigt. Glykolische Lösungen des Esters II, die praktisch keine Ester III enthalten, lassen sich nur mit sehr großen molaren Überschüssen an Glykol IV, beispielsweise von mehr als 200 Mol je mol Dicarbonsäure herstellen. Praktisch wird man stets Ester II und Ester III nebeneinander vorliegen haben. Beispielsweise kann man mit gutem Erfolg Lösungen der Ester II und sowie III bis V einsetzen, in denen der Ester II in Mengen von 70 bis 95 Gew.-% vorliegt, bezogen auf die Summe der Ester II und III bis V. Solche Lösungen entstehen, wenn man mit 5 bis 20 Mol Diglykol je Mol Dicarbonsäurebaustein in den Estern II und III bis V arbeitet.

Wenn man die glykolischen Lösungen der Ester II und III bis V auf die beschriebene Weise herstellt, enthalten sie in der Regel die erforderlichen Mengen Glykol VI. Wenn dies nicht Der Fall ist oder eine an sich ausreichende Menge zwecks Optimierung noch vergrößert werden soll, wird weiteres Glykol zugesetzt. Eine Optimierung kann beispielsweise erfolgen, wenn bei der Herstellung der glykolischen Lösung der Ester II und III bis V im Interesse einer hohen Raum-Zeit-Ausbeute weniger Glykol eingesetzt wurde, als für die Cyclisierungsreaktion wünschenswert ist. Vorzugsweise liegt das Glykol in der 2- bis 20-fachen molaren Menge vor, bezogen auf die Dicarbonsäurebausteine der Ester II und III bis V.

Als Katalysatoren. die sowohl für die Herstellung der glykolischen Lösungen der Ester II und III bis V als auch für die erfindungsgemäße Cyclisierungsreaktion brauchbar sind, können die üblichen sauren oder basischen Veresterungskatalysatoren dienen, die bekanntlich zugleich Umesterungskatalysatoren sind. Geeignete Katalysatoren sind z.B. unter den Verfahrensbedingungen hinreichend stabile Säuren, wie Schwefelsäure. Natriumhydrogensulfat, Phosphorsäure und Sulfonsäuren; weiterhin Alkalimetalle und Alkalialkoholate,: sowie Magnesium-, Mangan-, Cadmium-, Eisen-, Cobalt-, Zinn-, Blei-, Aluminium- und Titanverbindungen. Bevorzugt werden homogen gelöste Katalysatoren vom Typ einer Lewis-Säure verwendet. Auch die in der gleichzeitig anhängigen deutschen Patentanmeldung (O.Z. 5276) beschriebenen Eisen(III)-komplexe lassen sich mit Vorteil als Katalysatoren für die vorliegende Erfindung verwenden.

Die für die Herstellung der glykolischen Lösungen der Edukte II und III verwendeten Katalysatoren können im allgemeinen gleich in der Lösung verbleiben, da sie, wie gesagt, zugleich brauchbare Katalysatoren für die erfindungsgemäße Cyclisierungsreaktion sind. Dies gilt besonders dann. wenn die Katalysatoren homogen gelöst sind. Allerdings kann zusätzlich oder statt dessen Katalysator mit dem inerten hochsiedenden Reaktionsmedium eingebracht werden. Wenn man das Verfahren nach der Erfindung kontinuierlich durchführt, kann man dem zurückgeführten inerten hochsiedenden Medium, das bereits Katalysator enthält, weiteren Katalysator zufügen.

Es hat sich als zweckmäßig erwiesen, wenn in der Verdampfungszone pro Mol Dicarbonsäure-bis(glykol)ester II bzw. Dicarbonsäurebaustein in den Estern III bis V 0,01 bis 10 Gew.-% Katalysator vorliegen. Wenn man das Verfahren kontinuierlich durchführt, kann man die erwünschte Katalysatorkonzentration in der Verdampfungszone durch die Umlaufgeschwindigkeit des inerten hochsiedenden Mediums regeln. Bei hoher Umlaufgeschwindigkeit ist es möglich, hohe Konzentrationen an Katalysator in der Verdampfungszone bereitzustellen, ohne daß entsprechend große Mengen an Katalysator mit der glykolischen Lösung der Edukte II und III bis V zugeführt werden müssen. Dies ist eine Vorteil des erfindungsgemäßen Verfahrens in seiner kontinuierlichen Ausführungsform.

Geeignete inerte hochsiedende. d.h. unter Atmosphärendruck >400°C siedende Medien sind z.B. Polyalkylenglykoldialkylether. Im einzelnen seien beispielsweise genannt: Polyethylenglykoldimethylether (PEG DME) 1000, 2000 oder 5000; Polyethylenglykoldiethylether (PEG DEE) 1000, 2000, 5000; entsprechende oder ähnliche Polypropylenglykoldimethyl- oder diethylether sowie die Dimethyl- oder Diethylether gemischter Polyethylenpropylenglykole mit Blockstruktur oder statistischer Verteilung. Die Zahlen bedeuten das Molekulargewicht.

Neben der Anwesenheit bestimmter molarer Mengen Glykol, bezogen auf den Ester II und die Dicarbonsäurebausteine in den Estern III bis V, ist auch die Gewichtsmenge an inertem hochsiedendem Medium, bezogen auf die Summe der Gewichtsmengen der Ester II und III bis V, ein wesentliches Merkmal der Erfindung. Als Ergebnis dieser Maßnahmen wird in nur einem Durchgang ein weitgehender Umsatz der Ester II und III bis V zu dem cyclischen Makroester I erreicht, so daß bei kontinuierlicher Durchführung des Verfahrens kaum eine Anreicherung an nichtflüchtigen, höhermolekularen oder hochpolymeren Produkten in dem im Kreis geführten Reaktionsmedium erfolgt. Die benötigte Menge des inerten, hochsiedenden Mediums richtet sich dabei nicht nur nach der in einer Zeiteinheit zugeführten Menge an Estern II und III bis V sowie Glykol IV, sondern auch nach der optimalen Verweilzeit in der Verdampfungszone, die u.a. von den apparativen Verhältnissen, insbesondere der Art des Verdampfers und der Umwälzleistung der Pumpe, sowie von der Temperatur in der Verdampfungszone abhängt. Auf jeden Fall müssen die relevanten Parameter so aufeinander abgestimmt werden, daß in der Verdampfungszone die 0,1 bis 20-fache, vorteilhaft die 1- bis 15-fache und insbesondere die 2- bis 10-fache Gewichtsmenge, bezogen auf die Summe der Gewichtsmengen der Ester II und III bis V, an inertem hochsiedendem Medium vorliegt. Ein größerer Verdünnungsgrad, d.h. eine mehr als 20-fache Menge an inertem hochsiedendem Medium, bringt keinen ökonomischen Vorteil, ist allerdings für den Reaktionsverlauf auch nicht nachteilig.

Als Verdampfer und zugleich Reaktoren eignen sich alle üblichen Verdampfer mit großer Oberfläche, wie Dünnschicht-, Fallfilm-, Rieselfilm- und Kurzwegverdampfer. Die erforderliche Wärmemenge kann in diesen Fällen vorteilhaft direkt über den Verdampfer eingebracht werden. Wird ein Kreislaufstrom des inerten hochsiedenden Mediums über einen Wärmetauscher geführt, so kann die Reaktion auch in einem Rieselbettreaktor stattfinden. Eine andere geeignete Variante ist ein Rührreaktor mit einem Festbettkatalysator, der mit einem üblichen Verdampfer mit großer Oberfläche über eine Kreislaufführung verbunden ist. Schließlich kann man auch das vorerhitzte, Edukt II bis V und Glykol VI sowie Katalysator enthaltende hochsiedende Reaktionsmedium in einen Verdampfer einsprühen, in dem ein erhitztes inertes Trägergas strömt und den makrocyclischen Ester I sowie überschüssiges Glykol VI austrägt, während das hochsiedende Reaktionsmedium aus dem Verdampfer flüssig abläuft. In geeigneten Verdampfern liegt das hochsiedende Reaktionsmedium, welches Edukt II bis V, Glykol VI, Katalysator und nach Fortschreiten der Reaktion auch makrocyclischen Ester I enthält, in dünner Schicht von weniger als 2 cm Dicke, vorteilhaft von weniger als 0.5 cm Dicke oder in Tropfenform vor, bietet also eine große, verdampfungsfördernde spezifische Oberfläche. Dementsprechend kurz sind die Verweilzeiten des hochsiedenden Reaktionsmediums, in dem sich die Umwandlung vom Edukt zum Produkt vollzieht. Großoberflächige Verdampfer gestatten daher bei für den jeweiligen makrocyclischen Ester geeigneten Reaktionstemperaturen einen mindestens 80-prozentigen. vorteilhaft mindestens 90-prozentigen Umsatz des Edukts innerhalb einer Verweilzeit von weniger als 5 Minuten, vorteilhaft von weniger als 2 Minuten. Meistens liegen die Verweilzeiten sogar im Sekundenbereich. Das Destillat trennt sich in allen Fällen in zwei Phasen, wobei der makrocyclische Ester I die obere und das Glykol VI die untere Phase darstellt.

Es ist überraschend, daß die erfindungsgemäße Reaktion gerade in einem Verdampfer mit großer Oberflache so gut gelingt, weil unter diesen Bedingungen das Glykol, dessen Anwesenheit in einem molaren Überschuß ein wichtiges Merkmal des Verfahrens nach-der Erfindung ist. als Stoff mit dem niedrigsten Siedepunkt aller im Reaktionsgemisch vorhandenen Komponenten besonders schnell aus dem Reaktionsgemisch entfernt wird.

Das erfindungsgemäße Verfahren zur Herstellung makrocyclischer Ester führt überraschend selbst dann noch zu hohen. nahezu quantitativen Ausbeuten, wenn neben dem monomeren Ester II höhermolekulare Ester III bis V in erheblichen Mengen vorliegen. Sollten sich aber nach längerer Zeit in dem im Kreis geführten inerten hochsiedenden Medium höherpolymere Ester ansammeln, so können auch diese durch kurzzeitig erhöhte Glykolzufuhr in makrocyclischen Ester I überführt werden. Alternativ kann man auch eine Teilmenge des mit höhermolekularen oder hochpolymeren Estern angereicherten inerten hochsiedenden Mediums bei der Herstellung der glykolischen Lösung der Ester II und III verwenden oder mitverwenden. Auch dabei findet ein Abbau zu Estern II und III bis V statt.

### Beispiel 1 bis 6

### Allgemeine Vorschrift

X kg/h Dicarbonsäurebisglykolester der allgemeinen Formel II mit m=11 und n=2 (Brassylsäure-bis-hydroxyethylester), die 0.12 Gew.-% des Mono-Na-Salzes des Fe(III)-Ethylendiamintetraessigsäure-Komple-xes enthalten, werden zusammen mit Y kg/h Ethylenglykol einem Reaktor zugeführt, in dem sich Polyethylenglykol(2000)dimethylether als hochsiedendes Medium befindet. Der Reaktor besteht aus einem beheizten Dünnschichtverdampfer mit ca. 1,5 m² Oberfläche und einer in den Heizkreislauf eingebundenen Sumpfvorlage mit einem Volumen von 50 l sowie einer Rückführung zum Kopf des Fallfilmverdampfers, der auf der Eingangsseite mit einem Wärmetauscher versehen ist. Der Dünnschichtverdampfer wird unter einem Druck p betrieben. Die Temperatur des Einlaufs am Eingang des Verdampfers ist Tₘₐₓ, die Temperatur des Verdampfers wird im Ablauf gemessen und als T_{V} bezeichnet. Der Rotor des Dünnschichtverdampfers wird mit der Umdrehungszahl U betrieben.

Die Brüden des Dünnschichtverdampfers werden über eine Brüdenrohr zu einem Kondensator geleitet. Das Kondensat gelangt in eine Vorlage der ein Trenngefäß nachgeschaltet ist. Aus dem Trenngefäß wird als obere Phase Ethylenbrassylat mit etwa 7 Gew-% Ethylenglykol abgezogen. Daraus wird durch einfache Destillation reines Ethylenbrassylat gewonnen. Die untere Phase besteht aus Ethylenglykol, in dem Spuren von Ethylenbrassylat enthalten sind. Sie wird zusammen mit frischen Ethylenglykol in das Verfahren zurückgeführt.

Die Einsatzmengen, Verfahrensbedingungen und Ergebnisse der Beispiele gehen aus der folgenden Tabelle hervor.

**Tabelle**

| Bsp. | X | Y | p | T_{V} | Tₘₐₓ | U | Ausbeute | |
|---|---|---|---|---|---|---|---|---|
| | (kg/h) | (kg/h) | (hPa) | (°C) | (°C) | (min⁻¹) | (kg/h) | (%) |
| 1 | 5 | 15 | 0,1 | 280 | 290 | 200 | 4,8 | 96 |
| 2 | 7 | 14 | 0.05 | 240 | 300 | 250 | 6,6 | 94 |
| 3 | 9 | 35 | 25 | 260 | 270 | 300 | 8,7 | 97 |
| 4 | 12 | 28 | 15 | 290 | 300 | 300 | 11,2 | 93 |
| 5 | 9 | 25 | 10 | 300 | 300 | 200 | 8,5 | 94 |
| 6 | 3 | 6 | 75 | 290 | 290 | 300 | 2,7 | 92 |

## Patentansprüche

1. Verfahren zur Herstellung von makrocyclischen Estern der allgemeinen Formel 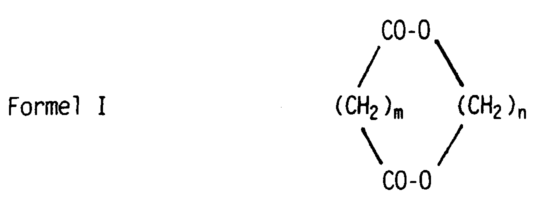 in der m die Bedeutung einer ganzen Zahl von 6 bis 14 hat und n für eine ganze Zahl von 2 bis 6 steht, durch Cyclisierung von Dicarbonsäure-bis(glykol)estern der allgemeinen Formel
HO-(CH₂)ₙ-O-CO-(CH₂)ₘ-CO-O-(CH₂)ₙ-OH Formel II
in der m und n die oben angegebenen Bedeutungen haben, wobei man
(a) einen Dicarbonsäure-bis(glykol)ester II.
(b) gegebenenfalls bis zu 100 Gew.-%, bezogen auf den Ester II, von hinsichtlich ihrer Bausteine dem Ester II entsprechenden Estern der allgemeinen Formeln
H[O-(CH₂)ₙ-O-CO-(CH₂)ₘ-CO]ₓ-O-(CH₂)ₙ-OH Formel III
H[O-(CH₂)n-O-CO-(CH₂)ₘ-CO]ₓ-OH Formel IV
und/oder
HO-CO-(CH₂)ₘ-CO-[O-(CH₂)ₙ-O-CO-(CH₂)ₘ-CO]ₓ-OH, Formel V
in denen m und n die zuvor angegebenen Bedeutungen haben und x für eine ganze Zahl >1 steht, sowie
(c) ein Glykol der allgemeinen Formel
HO-(CH₂)ₙ-OH Formel VI
in der n denselben numerischen Wert wie in den Formeln I bis V hat, in der 1- bis 50-fachen molaren Menge, bezogen auf den Ester II und die Dicarbonsäurebausteine der Esters III bis V, und
(d) ein inertes hochsiedendes Reaktionsmedium in der 0,1 bis 20-fachen Gewichtsmenge, bezogen auf die Summe der Gewichtsmengen der Ester II und III bis V, in Gegenwart
(e) eines Katalysators
in einem Verdampfer mit großer Oberfläche auf Temperaturen von 150 bis 350°C bei einem vermindertem Druck von etwa 0,1 bis etwa 500 mbar erhitzt, wodurch unter Abspaltung von Glykol der makrocyclische Ester I entsteht, der zusammen mit Glykol IV abdestilliert und durch Kondensation gewonnen wird.

2. Verfahren nach Anspruch 1, dadurch gekennzeichnet, daß das Verfahren kontinuierlich durchgeführt wird, wobei das inerte hochsiedende Medium, das den Katalysator und nicht umgesetzte Ester II und III enthält, im Kreis geführt und dem Kreislauf frischer Ester II und gegebenenfalls III bis V sowie Glykol VI zugesetzt wird.

3. Verfahren nach Anspruch 1 oder 2, dadurch gekennzeichnet, daß das Glykol in der 2- bis 20-fachen molaren Menge, bezogen auf die Dicarbonsäurebausteine der Ester II bis V, eingesetzt wird.

4. Verfahren nach einem der Ansprüche 1 bis 3, dadurch gekennzeichnet, daß die Temperatur 180 bis 300°C beträgt.

5. Verfahren nach einem der Ansprüche 1 bis 3, dadurch gekennzeichnet, daß die Temperatur 200 bis 280°C beträgt.

6. Verfahren nach einem der Ansprüche 1 bis 5, dadurch gekennzeichnet, daß der Druck 0,5 bis 100 mbar beträgt.

7. Verfahren nach einem der Ansprüche 1 bis 6, dadurch gekennzeichnet, daß als inertes hochsiedendes Medium ein oder mehrere Lösemittel aus der Gruppe der inerten höheren Glykoldialkylether und Polyalkylenglykoldialkylether verwendet wird bzw. werden.

8. Verfahren nach einem der Ansprüche 1 bis 7, dadurch gekennzeichnet, daß das inerte hochsiedende Medium in der Reaktionszone in der 1- bis 15-fachen Gewichtsmenge, bezogen auf die Summe der Gewichtsmengen der Ester II bis V, vorliegt.

9. Verfahren nach einem der Ansprüche 1 bis 7, dadurch gekennzeichnet, daß das inerte hochsiedende Medium in der Reaktionszone in der 2- bis 10-fachen Gewichtsmenge, bezogen auf die Summe der Gewichtsmengen der Ester II bis V, vorliegt.

10. Verfahren nach einem der Ansprüche 1 bis 9, dadurch gekennzeichnet, daß die Ester II und III bis V sowie das Glykol VI in Form einer glykolischen Lösung der beiden Ester eingesetzt werden, die durch Veresterung der Dicarbonsäure mit überschüssigem Glykol, durch Umesterung eines Dicarbonsäuredialkylesters mit Alkylresten mit 1 bis 6 Kohlenstoffatomen mit überschüssigem Glykol oder durch Depolymerisation von hochpolymeren Estern III bis V (x = >10) mit überschüssigem Glykol entstanden ist.

11. Verfahren nach einem der Ansprüche 1 bis 10, dadurch gekennzeichnet, daß man als Katalysator (e) einen der üblichen sauren oder basischen Veresterungs- oder Umesterungskatalysatoren verwendet.

12. Verfahren Anspruch 11, dadurch gekennzeichnet, daß man als Katalysator (e) Schwefelsäure, Phosphorsäure oder eine Sulfonsäure verwendet.

13. Verfahren Anspruch 11, dadurch gekennzeichnet, daß man als Katalysator (e) ein Alkalimetall oder -alkoholat verwendet.

14. Verfahren Anspruch 11, dadurch gekennzeichnet, daß man als Katalysator (e) eine oder mehrere Magnesium-, Mangan-, Cadmium-, Eisen-, Cobalt-, Zinn-, Blei-, Aluminium- oder Titanverbindungen verwendet.

15. Verfahren nach Anspruch 10, dadurch gekennzeichnet, daß für die Veresterung bzw. Umesterung derselbe Katalysator verwendet wird, der später als Katalysator (e) dient.

16. Verfahren nach einem der Ansprüche 2 bis 15, dadurch gekennzeichnet, daß man dem im Kreis geführten inerten hochsiedendem Medium Katalysator (e) zusetzt.

17. Verfahren nach einem der Ansprüche 1 bis 15, dadurch gekennzeichnet, daß man als Verdampfer mit großer Oberfläche einen wie Dünnschicht-, Fallfilm- und Kurzwegverdampfer verwendet.

18. Verfahren nach einem der Ansprüche 1 bis 15, dadurch gekennzeichnet, daß man als Verdampfer mit großer Oberfläche das Rieselbett einer Kolonne verwendet.
